# EUROPEAN PATENT APPLICATION

(11) **EP 4 464 322 A1**
(43) Date of publication of application: **20.11.2024**
(21) Application number: 23740337.3
(22) Date of filing: 13.01.2023
(51) Int. Cl.: A61K 31/7004, A23L 33/125, A61K 8/60, A61P 1/18, A61P 25/00, A61P 35/00, A61P 43/00, A61Q 19/08, C12N 5/00

(54) **CELL PROLIFERATION RETARDANT, COSMETIC CONTAINING SAME, AND CELL PROLIFERATION RETARDATION METHOD**

(30) Priority: 14.01.2022 JP 2022004569
(71) Applicant: National University Corporation Kagawa University, Takamatsu-shi, Kagawa 760-8521 (JP); SETOLAS Holdings, Inc., Takamatsu-shi, Kagawa 760-0026 (JP)
(72) Inventor: NISHIYAMA, Akira, Kita-gun, Kagawa 761-0793 (JP); MIYAKE, Keisuke, Kita-gun, Kagawa 761-0793 (JP); YOSHIHARA, Akihide, Takamatsu-shi, Kagawa 760-8521 (JP); IZUMORI, Ken, Takamatsu-shi, Kagawa 760-8521 (JP)
(74) Representative: AWA Sweden AB
(86) International application number: PCT/JP2023/000825
(87) International publication number: WO 2023/136329

(57) **Abstract**

The present invention provides: a cell proliferation retardant which contains D-allose and/or a derivative thereof and/or a mixture of D-allose and a derivative thereof as an active ingredient and which prolongs a period retaining in the G0/1 phase in the cell cycle; and a cosmetic which contains the cell proliferation retardant and retards the aging of a cell. The present invention also provides a method for retarding cell proliferation in a subject who needs the retardation of cell proliferation, the method comprising administering a cell proliferation retardant containing D-allose and/or a derivative thereof and/or a mixture of D-allose and a derivative thereof as an active ingredient to the subject to prolong a period retaining in the G0/1 phase in the cell cycle.

## Description

### FIELD

The present invention relates to an agent for delaying cell proliferation which contains D-allose and/or a derivative thereof, and/or a mixture thereof as an active ingredient, and to a cosmetic comprising it and to a method for delaying cell proliferation.

### BACKGROUND

In recent years, bacteria and yeast have been discovered that have populations with slow proliferation rates, whose cell cycles slow down overall without completely stopping (stasis) (NPLs 1 and 2). This cellular phenomenon is referred to as "cell slow-cycling", as a cell function that has been acquired during the course of biological evolution in order to survive in harsh environments, and the presence of similar cell populations has been confirmed in cancer as well (NPL 3). While the phenomenon whereby cells slow down their cell cycle without stopping (known as "cell slow-cycling") is thought to be a latent ability within cells that was acquired during the course of biological evolution, it is observed only under extreme conditions and is not a normally occurring phenomenon (NPL 4).

In recent years it has been reported that D-allose, known as a rare sugar, can inhibit proliferation of cancer cell lines, and that thioredoxin-interacting protein (TXNIP) specifically induced by D-allose, has an effect of "arresting" the cell cycle by stabilizing p27^{kip1} protein (a cell cycle protein) (NPL 5), or that D-allose has an effect of delaying the "G2 phase" of the cell cycle (PTL 1).

Another known rare sugar, allitol, is known to have a fat accumulation-inhibiting effect (PTL 2).

### [CITATION LIST]

### [PATENT LITERATURE]

[PTL 1] Japanese Patent Publication No. 5330976
[PTL 2] International Patent Publication No. WO2020/195106

### [NON PATENT LITERATURE]

[NPL 1] Balaban NQ., et al., Bacterial persistence as a phenotypic switch. Science. 2004, Sep 10; 305(5690): 1622-5.
[NPL 2] van Dijk D., et al., Slow-growing cells within isogenic populations have increased RNA polymerase error rates and DNA damage. Nat. Commun. 2015 Aug 13; 6:7972.
[NPL 3] Roesch A, et al., A temporarily distinct subpopulation of slow-cycling melanoma cells is required for continuous tumor growth. Cell. 2010 May 14; 141(4):583-94.
[NPL 4] Min M, Spencer SL. Spontaneously slow-cycling subpopulations of human cells originate from activation of stress-response pathways. PLoS Biol. 2019 Mar 13; 17(3):e3000178.
[NPL 5] Hirata Y, et al., Analysis of the inhibitory mechanism of D-allose on MOLT-4F leukemia cell proliferation. J. Biosci. Bioeng. 2009 May; 107(5):562-8

### SUMMARY

### [TECHNICAL PROBLEM]

It is an object of the present invention to provide an agent for delaying cell proliferation which is based on a novel mechanism, as well as a cosmetic comprising it, and a method for delaying cell proliferation.

### [SOLUTION TO PROBLEM]

As a result of much diligent research, the present inventors have completed this invention upon newly finding that the rare sugar D-allose or its derivative has an effect of lengthening the period in which cells remain in the G0/1 phase of the cell cycle.

Although the rare sugar D-allose has been reported to arrest the cell cycle via TXNIP and to delay the G2 phase of the cell cycle (PTL 1 and NPL 5), while the D-allose derivative allitol has been reported to have an anti-obesity effect (PTL 2), it has not been reported that both of these can lengthen the period in which cells remain in the G0/1 phase of the cell cycle, and particularly that they can lengthen the period in which cells remain in the G0/1 phase of the cell cycle without temporarily arresting the cell cycle, but this has been demonstrated for the first time by the present inventors.

Specifically, the present invention encompasses the following inventions.

[1] An agent for delaying cell proliferation, comprising D-allose and/or a derivative thereof, and/or a mixture thereof as an active ingredient, wherein the agent lengthens the period in which cells remain in the G0/1 phase of the cell cycle.
[2] The agent for delaying cell proliferation according to [1] above, wherein the D-allose derivative is allitol.
[3] The agent for delaying cell proliferation according to [1] or [2] above, which does not arrest the cell cycle.
[4] The agent for delaying cell proliferation according to any one of [1] to [3] above, which inhibits activity of the glycolysis in cells.
[5] The agent for delaying cell proliferation according to any one of [1] to [4] above, which delays proliferation of cancer cells.
[6] The agent for delaying cell proliferation according to any one of [1] to [5] above, which delays proliferation of pancreatic cancer cells or brain tumor cells.
[7] The agent for delaying cell proliferation according to any one of [1] to [4] above, which delays senescence of cells.
[8] A cosmetic for delaying senescence of cells, comprising an agent for delaying cell proliferation according to any one of [1] to [4] above.
[9] A method for delaying cell proliferation in a subject in need thereof, comprising administrating an agent for delaying cell proliferation comprising D-allose and/or a derivative thereof, and/or a mixture thereof as an active ingredient, to the subject, to lengthen the period in which cells remain in the G0/1 phase of the cell cycle.
[10] The method according to [9] above, wherein the D-allose derivative is allitol.

### [ADVANTAGEOUS EFFECTS OF INVENTION]

According to the present invention it has become possible to specifically lengthen the period during which cells remain in the G0/1 phase of the cell cycle, without arresting the cell cycle, and to reproduce the phenomenon of "cell slow-cycling" which has previously been thought to be observed only under extreme conditions. The invention with this effect has a wide range of potential applications including preventing age-related changes in various tissues and organs, inhibiting cancer growth and being usable as an anti-aging agent for skin (for example, a cosmetic).

### BRIEF DESCRIPTION OF DRAWINGS

Fig. 1 shows evaluation of cell proliferation ability using the human brain tumor cell lines U251 and U87. The cell proliferation ability in medium alone (control) and 48 hours after administration of D-glucose or D-allose was compared by WST-1 assay.
Fig. 2 shows evaluation of apoptosis using the human brain tumor cell lines U251 and U87. The proportion of apoptotic cells in medium alone (control) and 48 hours after administration of D-glucose or D-allose was compared by flow cytometry analysis of the cells stained with annexin V
Fig. 3 shows cell cycle evaluation using the human brain tumor cell lines U251 and U87. The proportion of cells in each cell cycle in medium alone (control) and 48 hours after administration of D-glucose or D-allose was compared by flow cytometry analysis.
Fig. 4-1 shows examination using the cell proliferation monitoring reagent CytoTell^{™} fluorescent probe.
Fig. 4-2 shows examination using the cell proliferation monitoring reagent CytoTell^{™} fluorescent probe.
Fig. 5 shows evaluation of cell proliferation ability for cultured human pancreatic cancer cells PK-1. The cell proliferation ability in medium alone (control) and 48 hours after administration of D-glucose or D-allose was compared by WST-1 assay.
Fig. 6 shows evaluation of apoptosis for cultured human pancreatic cancer cells PK-1. The proportion of apoptotic cells in medium alone (control) and 48 hours after administration of D-glucose or D-allose was compared by flow cytometry analysis of the cells stained with annexin V.
Fig. 7 shows evaluation of the cell cycle for cultured human pancreatic cancer cells PK-1. The proportion of cells in each cell cycle in medium alone (control) and 48 hours after administration of D-glucose or D-allose was compared by flow cytometry analysis.
Fig. 8 shows evaluation of the cell cycle for cultured human pancreatic cancer cells PK-1. Culturing was carried out for 12 hours in the presence of 50 nM nocodazole, and the cell cycle was arrested. The nocodazole was removed to restart the cell cycle, while administering medium alone (control) or D-glucose or D-allose (50 mM), after which samples were periodically taken and the cell cycle ratio was analyzed by flow cytometry.

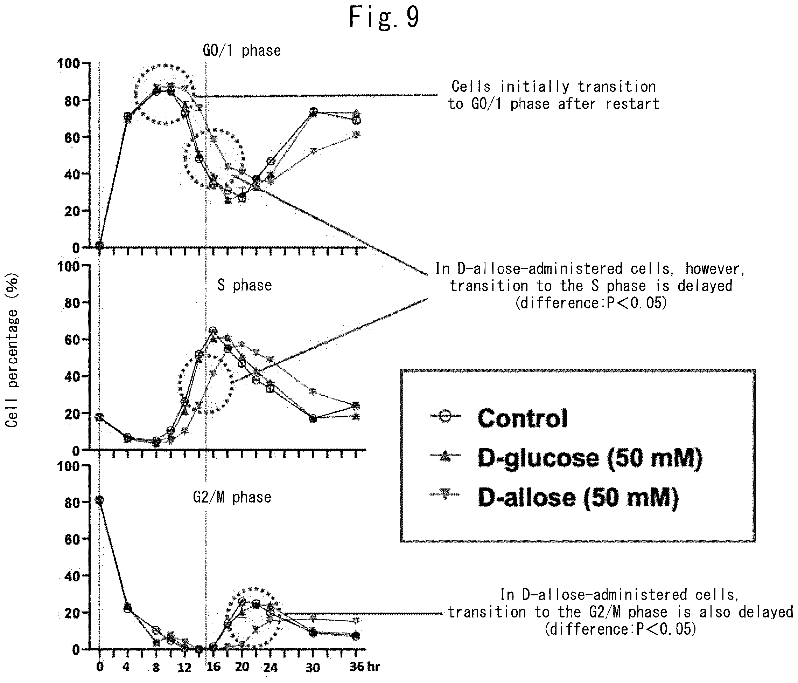

Fig. 9 shows delaying of the cell cycle transitioning from the G0/1 phase to the S phase. Shown is the cell cycle ratio (G0/1 phase, S phase and G2/M phase) for cultured human pancreatic cancer cells PK-1 cultured with the schedule in Fig. 8.
Fig. 10 shows evaluation of the glycolysis using a flux analyzer system.
Fig. 11 shows evaluation of cell proliferation ability for cultured human pancreatic cancer cells PK-1. The cell proliferation ability in medium alone (control) and 48 hours after administration of D-mannitol or allitol was compared by WST-1 assay.
Fig. 12 shows evaluation of apoptosis for cultured human pancreatic cancer cells PK-1. The proportion of apoptotic cells in medium alone (control) and 48 hours after administration of D-mannitol or allitol was compared by flow cytometry analysis of the cells stained with annexin V.

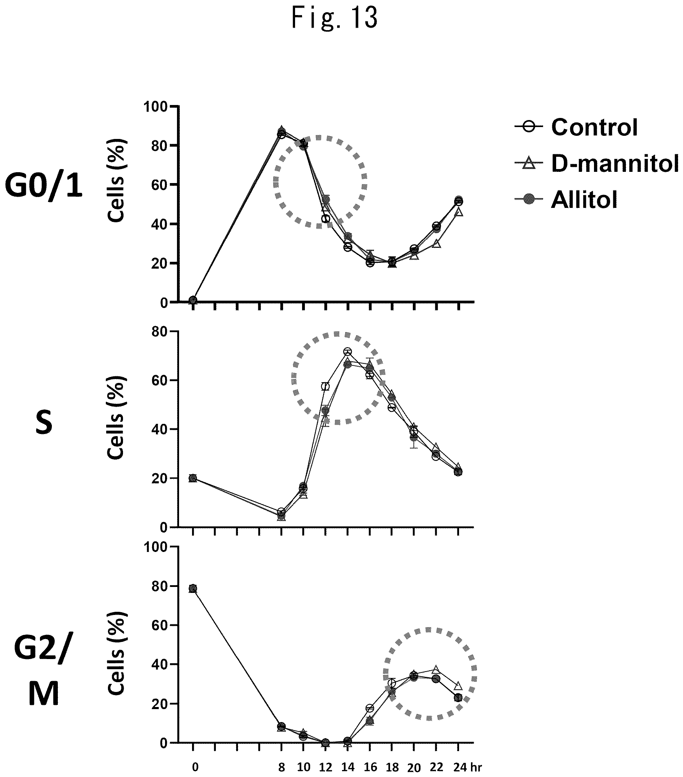

Fig. 13 shows delaying of the cell cycle transitioning from the G0/1 phase to the S phase. Culturing was carried out according to the schedule in Fig. 8, except for administering D-mannitol or allitol instead of D-glucose or D-allose. Each graph shows cell cycle ratios (G0/1 phase, S phase and G2/M phase) for cultured human pancreatic cancer cells PK-1.

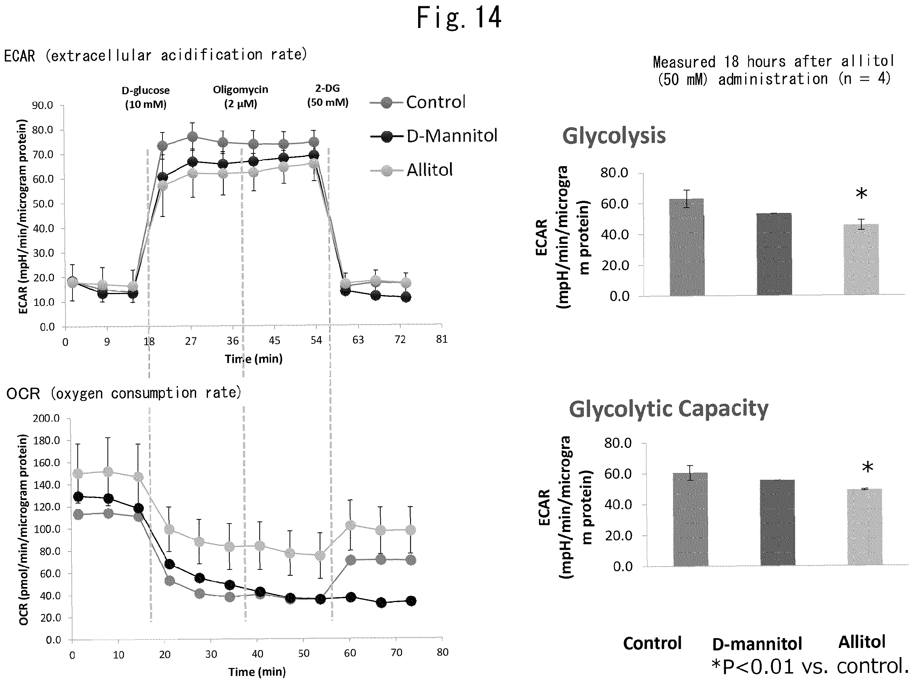

Fig. 14 shows evaluation of the glycolysis using a flux analyzer system.
Fig. 15 shows the results of principal component analysis based on metabolome analysis of substances associated with central energy metabolism in human pancreatic cancer cells PK-1 and PANC-1, cultured either without administration of sugar (control), or with addition of D-glucose or D-allose.
Fig. 16 shows the results of heat map analysis based on metabolome analysis of substances associated with central energy metabolism in human pancreatic cancer cells PK-1, cultured either without administration of sugar (control), or with addition of D-glucose or D-allose.
Fig. 17 shows the results of heat map analysis based on metabolome analysis of metabolites associated with the glycolysis in human pancreatic cancer cells PK-1 and PANC-1, cultured either without administration of sugar (control), or with addition of D-glucose or D-allose.
Fig. 18 shows the results of heat map analysis based on metabolome analysis of metabolites associated with gluconeogenesis, the polyol pathway and the pentose phosphate pathway (PPP) in human pancreatic cancer cells PK-1 and PANC-1, cultured either without administration of sugar (control), or with addition of D-glucose or D-allose.
Fig. 19 shows the results of heat map analysis based on metabolome analysis of metabolites associated with TCA cycle in human pancreatic cancer cells PK-1 and PANC-1, cultured either without administration of sugar (control), or with addition of D-glucose or D-allose.
Fig. 20 shows the results of pathway map analysis based on metabolome analysis of substances associated with central energy metabolism in human pancreatic cancer cells PK-1, cultured either without administration of sugar (control), or with addition of D-glucose or D-allose. Substance names in red (T): Substances increased by addition of D-allose. Substance names in blue (>k): Substances decreased by addition of D-allose. Substance names in black: Substances unmeasured or with no change by addition of D-allose.
Fig. 21 shows fluorescent microscope photographs of human pancreatic cancer cells PK-1 at 24 hours after transfection with tFucci(CA)5. G1 phase: red, S phase: green, G2/M phase: yellow-orange. NEB: nuclear membrane breakdown, NER: nuclear membrane regeneration.
Fig. 22 shows fluorescent microscope photographs of human pancreatic cancer cells PK-1 at 0 to 84 hours after transfection with tFucci(CA)5, either without addition of sugar (control) or after addition of D-allose. G1 phase: red, S phase: green, G2/M phase: yellow-orange. The "X" symbols in the photographs are cells derived from other cells not appearing at 0 hours.

### DESCRIPTION OF EMBODIMENTS

Embodiments for carrying out the invention will be described in detail below, with the understanding that the technical scope of the invention is not limited only to these embodiments. The prior art documents cited herein are incorporated in their entirety by reference throughout the present specification.

One embodiment of the invention provides an agent for delaying cell proliferation, comprising D-allose and/or a derivative thereof, and/or a mixture thereof as an active ingredient, and wherein the agent lengthens the period in which cells remain in the G0/1 phase of the cell cycle. The invention may be provided as a drug or it may be provided as a food. When the invention is provided as a food it may be a health functional food (such as a specified health food or nutritional function food), or a dietary supplement, for example.

Another embodiment of the invention provides a method for delaying cell proliferation in a subject in need thereof, comprising administrating an agent for delaying cell proliferation comprising D-allose and/or a derivative thereof, and/or a mixture thereof as an active ingredient, to the subject, to lengthen the period in which cells remain in the G0/1 phase of the cell cycle.

The present invention has been completed based on the new finding that the rare sugar D-allose and/or a derivative thereof, and/or a mixture thereof, has a hitherto unknown effect of lengthening the period in which cells remain in the G0/1 phase of the cell cycle.

As used herein, "cell slow-cycling" refers to a state in which the cell cycle progresses more slowly than conventional cells but without arresting the cell cycle ("cell cycle arrest"), and for example, cells cultured in medium comprising D-allose and/or a derivative thereof, and/or a mixture thereof, specifically lengthen the cell cycle, and particularly the period during which cells remain in the G0/1 phase of the cell cycle, thus lengthening the cell cycle, compared to cells cultured in medium without D-allose and/or a derivative thereof and/or a mixture thereof as according to the present invention.

The term "cell cycle" used herein refers to the cycle of events constituting cell division in eukaryotes, which includes mitosis, cytokinesis and interphase. The cell cycle in somatic cells during proliferation has historically been divided into the mitotic phase (M phase) and the interphase, but as DNA synthesis has been found to take place during part of the interphase, a synthesis phase (the S phase) has been inserted, and adding the M phase to the G1 phase, S phase and G2 phase results in a total of 4 phases of division normally considered to constitute the cell cycle. According to one aspect, therefore, the "cell cycle" is the cycle in which genetic information that has doubled during the S phase is equally distributed in the M phase, for replication of the cell. For the purpose of the invention, the time required for each phase of a single cell cycle can be calculated by a publicly known method, such as by direct observation of living cells, or measurement of the mitotic index, introduction by an autoradiographic method, or measurement of the DNA content in each cell using a cell sorter. Most cell types with arrested growth break out of the cell cycle during the G1 phase and transition to the G0 phase.

The "G0 phase" as referred to herein is the period in which a cell has stopped progressing through the cell cycle while in the G1 phase. Stopping progression through the cell cycle may occur during the G1 phase, G2 phase or M phase, but the G0 phase is generally considered to be when the cell has broken out of the cell cycle in the G1 phase, and this definition is also used herein throughout. Growth arrest by differentiation or senescence of a cell is a typical example of the G0 phase. Leaving or entering the G0 phase and G1 phase is considered to occur before a checkpoint in the G1 phase.

The major factors conventionally known to control progression of the cell cycle are CDK, cyclin and CDK inhibitor (CKI). CDK is a catalytic subunit of protein kinase. It is also considered to be a device driving cell cycle progression. Regulating factors for CDK include cyclin as an activating factor and CKI as an inhibiting factor. CDK and cyclin constitute different protein families, which promote cell cycle progression in different periods depending on the combination of subtypes. Typical examples of mammalian somatic cells include cyclin B-Cdc2(CDK1) complex (Cdc2 kinase) which promotes M phase progression, cyclin D-CDK4 complex and cyclin E-CDK2 complex which promote G1 phase progression, and cyclin A-CDK2 complex which promotes S phase progression. CKI is thought to suppress progression to the G1 phase and S phase and promote withdrawal into the G0 phase.

The phrase "period during which cells remain in the G0/1 phase of the cell cycle" as used herein means the period from the start of the G1 phase (i.e. the end of the M phase) up until the start of the S phase (i.e. the start of DNA synthesis), and/or the period at which a cell in the G0 phase enters the cell cycle (i.e. the cell cycle returns from the G0 phase to the G1 phase) until start of the S phase. The present inventors have found that D-allose can specifically lengthen the period during which cells remain in the G0/1 phase of the cell cycle, without arresting the cell cycle. As the period during which cells remain in the G0/1 phase of the cell cycle is specifically lengthened, this delays subsequent transition to the S phase, and further delays subsequent transition to the G2/M phase.

According to one aspect, the invention exhibits an effect of inhibiting activity of the glycolysis in cells. As used herein, "glycolysis" refers to a biochemical reaction pathway in the body (in cells) in which glucose is decomposed (catabolized) to organic acids such as pyruvic acid, being a metabolic process for conversion of the high bond energy in glucose to a form that can be used by the organism, and it has the same meaning as the common usage of the term. While being a representative example of a metabolic system that can function in an anaerobic state, it also functions as part of aerobic respiration in which the reducing power and pyruvic acid are transferred to the electron transfer system and citric acid cycle.

Glycolysis activity can be evaluated by measuring intracellular activity. Without being limitative, glycolysis activity can be evaluated, for example, by measuring the cellular oxygen consumption rate (OCR value) or extracellular acidification rate (ECAR value), or both the OCR and ECAR values (see Jing Zhang, and Qing Zhang, Using Seahorse Machine to Measure OCR and ECAR in Cancer Cells, Methods Mol Biol. 2019; 1928:353-363). As used herein, the phrase "inhibits glycolysis activity" may mean, for example, significantly lowering the extracellular acidification rate (ECAR value) compared to a control (for example, cells cultured in the absence of D-allose and/or allitol), or maintaining a significantly high state for the oxygen consumption rate (OCR value) compared to a control (for example, cells cultured in the absence of D-allose and/or allitol).

Intracellular activity may be measured by non-invasive and highly sensitive periodic measurement of cells to determine their state of glycolytic and mitochondrial aerobic respiration, as the main energy metabolism pathway of the cells, using e.g., an XFe24 extracellular flux analyzer (24-well) by Agilent Technologies (previously Seahorse Bioscience). However, this device is only an example of one device that may be used to measure intracellular activity, without being limitative, and any other device and method may be used for measurement and evaluation of the intracellular activity.

According to one embodiment, the invention can exhibit an effect of lengthening the period in which cells remain in the G0/1 phase of the cell cycle, thus allowing it to delay growth of cancer cells. The cancer to which the invention may be applied may be, for example, leukemia (such as acute myelocytic leukemia, chronic myelogenous leukemia, acute lymphocytic leukemia and chronic lymphatic leukemia), malignant lymphoma (Hodgkin's lymphoma, non-Hodgkin lymphoma (such as adult T cell leukemia, follicular lymphoma and diffuse large B-cell lymphoma)), multiple myeloma, myelodysplastic syndrome, head and neck cancer, gastrointestinal cancer (such as esophageal cancer, esophageal adenocarcinoma, stomach cancer, colorectal cancer, colon cancer and rectum cancer), liver cancer (such as hepatocellular carcinoma), gallbladder/cholangiocarcinoma, biliary tract cancer, pancreatic cancer, thyroid cancer, lung cancer (such as non-small-cell lung carcinoma (including squamous epithelium non-small-cell lung carcinoma and non-squamous non-small cell lung carcinoma) and small-cell lung cancer), breast cancer, ovarian cancer (such as serous ovarian cancer), cervical cancer, uterine cancer, endometrial cancer, vaginal cancer, vulvar cancer, renal carcinoma (such as renal cell carcinoma), urothelial cancer (such as bladder cancer and upper urinary tract cancer), prostate cancer, testicular cancer (such as germ cell tumor), bone/soft tissue sarcoma, skin cancer (such as uveal melanoma, malignant melanoma and Merkel cell carcinoma), glioma, brain tumor (such as glioblastoma), pleural mesothelioma and cancer of unknown origin. The cancer cells to which the invention may be applied may be pancreatic cancer cells or brain tumor cells, for example.

According to one embodiment, since the invention can exhibit an effect of lengthening the period in which cells remain in the G0/1 phase of the cell cycle it may be used to delay cell senescence, and for example, it may be employed as a cosmetic to be used for anti-aging purposes, or as a protective agent for grafted tissue or organs, as well as for future foods or drugs that inhibit biological aging.

The D-allose to be used for the invention is in extremely low abundance in the natural world, compared to D-glucose (glucose) which is abundantly present. Of the monosaccharides that are the base units of sugars (a total of 34 monosaccharides with 6 carbon atoms (hexoses) exist, 16 of which are aldoses, 8 of which are ketoses and 10 of which are sugar alcohols), monosaccharides that are only present in trace amounts in the natural world (aldoses and ketoses) and their derivatives (sugar alcohols) are defined as "rare sugars", in contrast to "naturally-occurring monosaccharides", typically D-glucose (glucose), that are highly abundant in the natural world. The rare sugars that can be mass produced as of the current writing are D-psicose and D-allose. D-allose is the D-form of allose, classified as an aldose, and it is a hexose.

Previously disclosed methods for obtaining "D-allose" include a method of synthesis from D-psicose using L-rhamnose isomerase, and a method of reacting D-xylose isomerase with a D-psicose-containing solution, but the D-allose of the invention is not limited to these methods and may be obtained by any method of isomerization by chemical treatment. The D-psicose used as starting material for D-allose will usually be obtained by treating fructose with an enzyme (epimerase), but this is not limitative, and it can be obtained by a method using microorganisms that produce the enzyme, or it may be an extract from a natural product, or a substance that is present in a natural product, or a substance isomerized by a chemical treatment method. The method for purifying the D-psicose using an enzyme may be a publicly known method.

The D-allose may also be in the form of D-allose-containing syrup. A D-allose-containing syrup can be obtained by appropriately mixing common syrups (liquid sugar), but it is also sold in stores as a "food" such as the commercial product "Rare Sugar Sweet" (manufacturer: RareSweet Co., Ltd., vendor: Matsutani Chemical Industry Co., Ltd.), and is readily available.

The method of obtaining the D-allose-containing syrup may be, for example, reacting an alkali with a monosaccharide (D-glucose or D-fructose) to cause Lobry de Bruyn-Van Ekenstein transformation or retro-aldol reaction and subsequent aldol reaction (such reactions known as "alkali isomerization reaction"), the syrup containing the produced monosaccharides (including rare sugars) being generally referred to as "rare sugar-containing syrup", and D-glucose and/or D-fructose may be used as the starting material to obtain an alkali-isomerized syrup with a D-glucose and/or D-fructose content of 55 to 99 mass%. The product "Rare Sugar Sweet" is a syrup containing rare sugars obtained by the method disclosed in International Patent Publication No. WO2010/113785 with isomerized sugar as the starting material, and it was produced so as to contain primarily D-psicose and D-allose as the rare sugars. The rare sugars in the rare sugar-containing syrup obtained by this method are 0.5 to 17 mass% D-psicose and 0.2 to 10 mass% D-allose, as proportions with respect to the total sugars. A publication by Takahashi et al. (Ouyou Toushitsu Kagaku, Vol. 5, No. 1, 44-49(2015)) reports that it is a syrup containing 5.4 g/100 g D-psicose, 5.3 g/100 g D-sorbose, 2.0 g/100 g D-tagatose, 1.4 g/100 g D-allose and 4.3 g/100 g D-mannose.

The starting materials used to produce the rare sugar-containing syrup are starch, sugar, isomerized sugar, fructose and glucose. Isomerized sugar is widely considered to be a mixture of sugars with a main composition of D-glucose and D-fructose in a specified compositional ratio, and generally refers to syrup consisting mainly of glucose and fructose, obtained by glucose isomerase or alkali isomerization of a sugar solution composed mainly of glucose from hydrolysis of starch using an enzyme such as amylase or an acid. According to the JAS standard, the term "glucose-fructose syrup " is applied to a fructose content (percentage of fructose of the total sugars) of less than 50%, "fructose-glucose syrup" is applied for ≥50% and <90%, "high fructose syrup" is applied for ≥90%, and "sugar-containing fructose-glucose syrup" is applied for syrups with addition of sugar to glucose-fructose syrup in an amount not exceeding the glucose-fructose syrup, and the starting material for the rare sugar-containing syrup of the invention may employ any of these isomerized sugars.

For example, rare sugar-containing syrup obtained using D-fructose as the starting material contains 5.2% D-psicose, 1.8% D-allose, 15.0% glucose and 69.3% D-fructose. Rare sugar-containing syrup obtained using isomerized sugar as the starting material contains 3.7% D-psicose, 1.5% D-allose, 45.9% glucose and 37.7% D-fructose, or when D-glucose is used as the starting material it contains 5.7% D-psicose, 2.7% D-allose, 47.4% glucose and 32.1% D-fructose, although the sugar composition will differ depending on differences in the starting material and treatment method. D-allose separated and purified from such syrups may also be used, or the syrups may be used directly.

According to one aspect, the D-allose to be used for the invention may be D-allose and/or a derivative thereof, and/or a mixture thereof. Compounds whose molecular structure is altered from starting compounds by chemical reaction are generally referred to as derivatives of the starting compounds. Throughout the present specification, "D-allose derivative" is used to mean a compound obtained by converting the molecular structure of the D-allose starting compound by chemical reaction; and also includes compounds obtained by converting the molecular structure of an analog compound of D-allose (such as D-glucose) as the starting compound by chemical reaction, being a compound having the same structure as a compound obtained by converting the molecular structure of D-allose by chemical reaction (also referred to as "D-allose structural analog"). According to one embodiment, the "D-allose derivative" used for the invention may be one containing allitol, as a reduction product of D-allose.

The compound "allitol" (D-allo-hexitol, or (2S,3S,4R,5R)-hexane-1,2,3,4,5,6-hexaol) referred to herein is a rare sugar classified as a polyol (sugar alcohol). Allitol is a sugar alcohol of 6 carbon atoms obtained by reduction of D-psicose or D-allose, and while being almost absent in the natural world it is known as a component of Itea plants (genus Itea, scientific name: Itea spp.) (also known as "sweetspire" in some regions of the world) (PTL 2). The allitol to be used for the invention may therefore be allitol extracted from a plant body belonging to the genus Itea, or allitol produced by hydrogenation reaction from D-psicose in the presence of a catalyst containing a metal selected from among elements of Group 8 of the Periodic Table, with no limitation to these. The allitol to be used for the invention may be allitol and/or a derivative thereof, and/or a mixture thereof.

The D-allose derivative for the purpose of the invention may be a sugar alcohol in which the carbonyl group of D-allose is the alcohol group (i.e. allitol), uronic acid in which the hydroxymethyl group of D-allose is converted to a carboxy group, aldonic acid in which the formyl group at position 1 of D-allose is a carboxyl group, aldanic acid in which the formyl group at position 1 of D-allose and the hydroxymethyl group at the end of the main chain are both changed to carboxyl groups, or an amino sugar in which the alcohol group of D-allose is converted to an amino group.

According to another aspect, the D-allose derivative may be a D-allose derivative in which any hydroxyl group of D-allose (such as the hydroxyl group at position 2, position 3, position 4, position 5 and/or position 6) is replaced with a hydrogen atom, a halogen atom, an amino, carboxyl, nitro, cyano, lower alkyl, lower alkoxy, lower alkanoyl, lower alkanoyloxy or lower alkoxycarbonyl group, a mono- or di- lower alkyl substituted amino group, or an aralkyl, aryl or heteroaryl group.

According to one embodiment, "D-allose derivative" is used to mean a compound obtained by converting the molecular structure of the allitol starting compound by chemical reaction; and may also include compounds obtained by converting the molecular structure of an analog compound of allitol (such as D-mannitol) as the starting compound by chemical reaction, being a compound having the same structure as a compound obtained by converting the molecular structure of allitol by chemical reaction (also referred to as "allitol structural analog"). According to another aspect, the D-allose derivative may be one in which any hydroxyl group of allitol (such as the hydroxyl group at position 1, position 2, position 3, position 4, position 5 and/or position 6) is replaced with a hydrogen atom, a halogen atom, an amino, carboxyl, nitro, cyano, lower alkyl, lower alkoxy, lower alkanoyl, lower alkanoyloxy or lower alkoxycarbonyl group, a mono- or di- lower alkyl substituted amino group, or an aralkyl, aryl or heteroaryl group.

Halogen atoms are the atoms fluorine, chlorine, bromine and iodine. The alkyl portion of a lower alkyl, lower alkoxy, lower alkoxycarbonyl or mono- or di- lower alkyl substituted amino group is a straight-chain, branched or cyclic C1-C6 alkyl group, with specific examples including methyl, ethyl, propyl, isopropyl, butyl, isobutyl, sec-butyl, tert-butyl, pentyl, isopentyl, neopentyl, hexyl, isohexyl, cyclopropyl, cyclobutyl, 2-methylcyclopropyl, cyclopropylmethyl, cyclopentyl and cyclohexyl groups.

The lower alkanoyl portion of a lower alkanoyl or lower alkanoyloxy group is a straight-chain, branched or cyclic C1-C7 alkanoyl group, with specific examples including formyl, acetyl, propionyl, butyryl, isobutyryl, valeryl, isovaleryl, pivaloyl, hexanoyl, cyclopropylcarbonyl, cyclobutylcarbonyl, 2-methylcyclopropylcarbonyl and cyclohexylcarbonyl groups.

An aralkyl group is a C7-C20 aralkyl group, with specific examples including benzyl, phenethyl, α-methylbenzyl, benzhydril, trityl and naphthylmethyl groups.

An aryl group is a C6-C14 aryl group, with specific examples including phenyl and naphthyl groups.

A heteroaryl group is a C3-C8 heteroaryl group, which is a monocyclic, polycyclic or fused ring of 1 to 4 N, O or S atoms which may be the same or different, with specific examples including 2-pyridyl, 3-pyridyl, 4-pyridyl, 2-quinonyl, 3-quinonyl, 4-quinonyl, 5-quinonyl, 6-quinonyl, 7-quinonyl, 8-quinonyl, 2-indolyl, 3-indolyl, 4-indolyl, 5-indolyl, 6-indolyl, 7-indolyl, 2-furyl, 3-furyl, 2-thienyl, 3-thienyl, 2-pyrrolidyl, 3-pyrrolidyl, 2-imidazolyl, 4-imidazolyl, 5-imidazolyl, 2-oxazolyl, 4-oxazolyl, 5-oxazolyl, 2-thiazolyl, 4-thiazolyl and 5-thiazolyl groups.

According to one aspect, the D-allose derivative to be used for the invention may be a D-allose derivative such as 2-deoxy-D-allose, 5-deoxy-D-allose, 6-deoxy-D-allose or 3-deoxy-D-allose(3 -deoxy-D-glucose).

The "D-allose and/or a derivative thereof, and/or a mixture thereof" to be used for the invention is also interpreted as including pharmacologically acceptable salts and/or hydrates.

Throughout the present specification, the term "food" means food in general, but in addition to common foods including health foods it also includes health functional foods such as specified health foods and nutritional function foods, as well as dietary supplements (supplements and nutritional supplements), feeds and food additives. The composition for suppressing production of cytokines of the invention has the food compound D-allose as the active ingredient, and may be used as a sweetener, seasoning, food additive, food material, food or beverage, health food or beverage, pharmaceutical or quasi drug, or feed, being able to suppress production of cytokines in any of these forms.

According to one embodiment, the composition of the invention may be administered by any desired route of administration. Routes of administration include local administration (percutaneous, inhalation, enema, eye drop, ear drop, pernasal or intravaginal), enteral administration (oral, tubal or enteral), or parenteral administration (intravenous, transarterial, transdermal or intramuscular injection).

According to another embodiment, the dosage for D-allose and/or a derivative thereof, and/or a mixture thereof, as an agent for delaying cell proliferation of the invention, may be adjusted as appropriate to be a dosage at which the effect of the invention is exhibited, and for example, it may be administered at 1 mg/kg body weight/day to 1000 mg/kg body weight/day, with adjustment as appropriate depending on the age and symptoms. It may be administered at a dosage of 1 mg/kg body weight/day to 1000 mg/kg body weight/day, such as 10 mg/kg body weight/day to 800 mg/kg body weight/day or 50 mg/kg body weight/day to 500 mg/kg body weight/day, as an amount that can be ingested for transintestinal administration.

When the rare sugar D-allose and/or a derivative thereof, and/or a mixture thereof is used as a component of a food, an effective dose of the rare sugar D-allose and/or a derivative thereof, and/or a mixture thereof, can be safely ingested in the course of routine dietary habit. The rare sugar D-allose is an aldose and is therefore a highly safe compound for administration to humans.

Throughout the present specification, the term "drug" is used to include drugs and quasi drugs.

The agent for delaying cell proliferation of the invention may also be provided as a composition, wherein D-allose and/or a derivative thereof, and/or a mixture thereof is provided by formulation to include suitable additives such as excipients, stabilizers, preservatives, binders or disintegrators, selecting a suitable dosage form such as tablets, powder, granules, capsules, a solution, syrup, elixir or an oily or aqueous suspension.

For a solid formulation, the D-allose and/or a derivative thereof, and/or a mixture thereof, may further include pharmaceutically acceptable additives, and for example, fillers or extenders, binders, disintegrators, dissolution accelerators, moistening agents or lubricants may be selected as necessary for mixing and formulation. For a solid formulation, an excipient, disintegrator, binder and lubricant may also be added to and mixed with the D-allose and/or a derivative thereof, and/or a mixture thereof, and the mixture may then be compacted and molded. Lactose, starch and mannitol are commonly used as excipients. Calcium carbonate and carboxymethyl cellulose calcium are commonly used as disintegrators. Gum arabic, carboxymethyl cellulose and polyvinylpyrrolidone are used as binders. Talc and magnesium stearate are publicly known lubricants.

Tablets can be coated in a publicly known manner for masking or as an enteric-coated formulation. Ethyl cellulose and polyoxyethylene glycol may be used as coating agents.

According to one embodiment, in addition to the aforementioned drugs (pharmaceutical compositions), the composition of the invention may be provided as a food (such as a medical food, specified health food, health assisting food, health food, nutritional function food, supplement, dietary supplement or herbal tea). D-allose may be administered at a dosage of 1 mg/kg body weight/day to 1000 mg/kg body weight/day (for example, 50 mg to 50 g/day for a 50 kg adult).

When an agent of the invention is to be used as a cosmetic, it may be used in the form of a cosmetic water, latex, foundation, lipstick, lip cream, cleansing cream, massage cream, pack, hand cream, hand powder, body, shampoo, body, lotion, body, cream or bath cosmetic.

In addition to the aforementioned components, there may also be appropriately added, as necessary, components that are commonly used in external preparations for skin such as cosmetics, drugs or quasi drugs, including antioxidants, oils, ultraviolet protecting agents, surfactants, thickeners, alcohols, powder constituents, coloring materials, aqueous components, water and skin nutrient preparations, in ranges that do not interfere with the effect of the invention.

An agent of the invention is not particularly restricted so long as can be used as a cosmetic to be applied to the outer skin as a cosmetic, or used as a quasi drug, and the dosage form is also not restricted if it can be applied to skin and may be a dosage form such as a solution or a solubilized, emulsified or powder-dispersed form, or a water-oil two-layer system, water-oil-powder three-layer system, ointment, cosmetic water, gel or aerosol.

Subjects for which the invention may be used include, but are not particularly limited to, animals including humans (mammals such as humans, cows, pigs, dogs and cats, and birds such as chickens).

The present invention will now be explained in greater detail by Examples, with the understanding that the invention is not limited in any way by the Examples.

### EXAMPLES

### <Example 1: Analysis using cultured human brain tumor cells>

U251 cells or U87 cells, as cultured human brain tumor cells, were cultured for 48 hours without administration of a sugar (control) or with addition of D-allose or D-glucose at different concentrations (3, 5, 10, 30 or 50 mM), and then analyzed by WST-1 assay to compare the effects on cell proliferation.

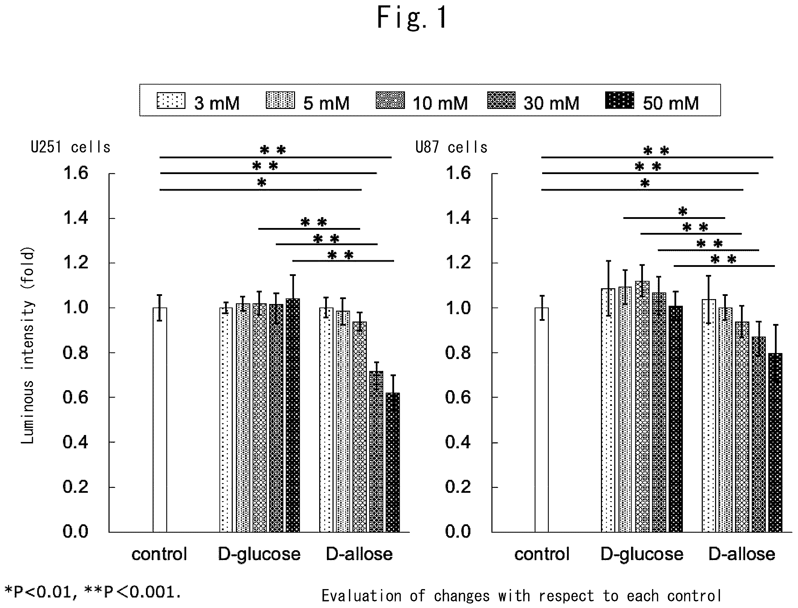

As a result, the rare sugar D-allose exhibited a dose-dependent cell growth inhibitory effect on U251 cells and U87 cells as cultured human brain tumor cells, as shown in Fig. 1.

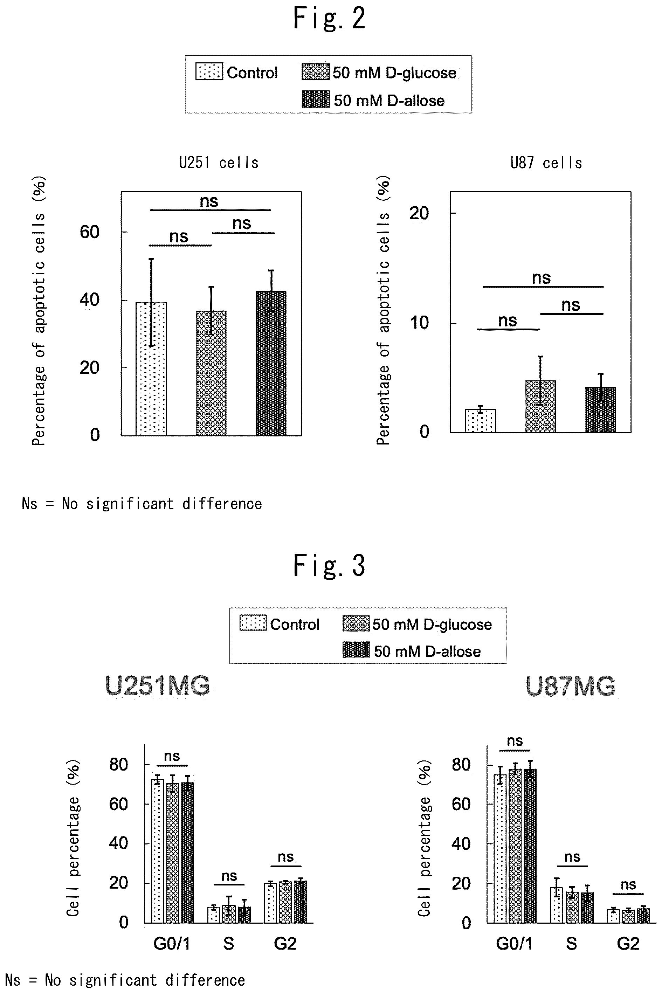

In order to elucidate the mechanism of the growth inhibition effect on brain tumor cells by D-allose, the cells were evaluated for apoptosis by flow cytometry after annexin V staining (Fig. 2).

As a result it was shown that apoptosis is not induced by doses of D-allose that induce a growth inhibition effect on brain tumor cells. In other words, the growth inhibition effect of D-allose on brain tumor cells was shown to be independent of apoptosis (Fig. 2).

Considering the possibility that growth inhibition of brain tumor cells may be caused by arrest of the cell cycle by D-allose, flow cytometry (FACS analysis) was carried out to identify changes in the cell cycle (see Yamaguchi F, Takata M, Kamitori K, et al. Rare sugar D-allose induces specific up-regulation of TXNIP and subsequent G1 cell cycle arrest in hepatocellular carcinoma cells by stabilization of p27kip1. Int J Oncol. 2008; 32(2):377-385.). Specifically, the cell cycle was evaluated by Propidium Iodide (PI)/RNase Staining Solution (Cell Signaling Technology). The cells were cultured for 24 hours using DMEM, 50 mM D-glucose or 50 mM D-allose. The cultured cells were then recovered using trypsin and washed twice with PBS, and then fixed with 70% ethanol overnight at -20°C. After washing with PBS, the cells were stained in a dark room at room temperature for 30 minutes using 500 µl of PI reagent, washed with PBS, and then monitored for luminescence at 617 nm by flow cytometry. The data were analyzed using Kaluza analysis software (Ver.1.2, Beckman Coulter), and the effects on the cell cycle were evaluated based on changes in the cell distribution rate at each phase of the cell cycle.

As a result, as shown in Fig. 3, administration of 50 mM D-allose which induces a growth inhibition effect on brain tumor cells did not induce arrest of the cell cycle of any of the brain tumor cells. In other words, the growth inhibition effect of D-allose on brain tumor cells was shown to be independent of cell cycle arrest.

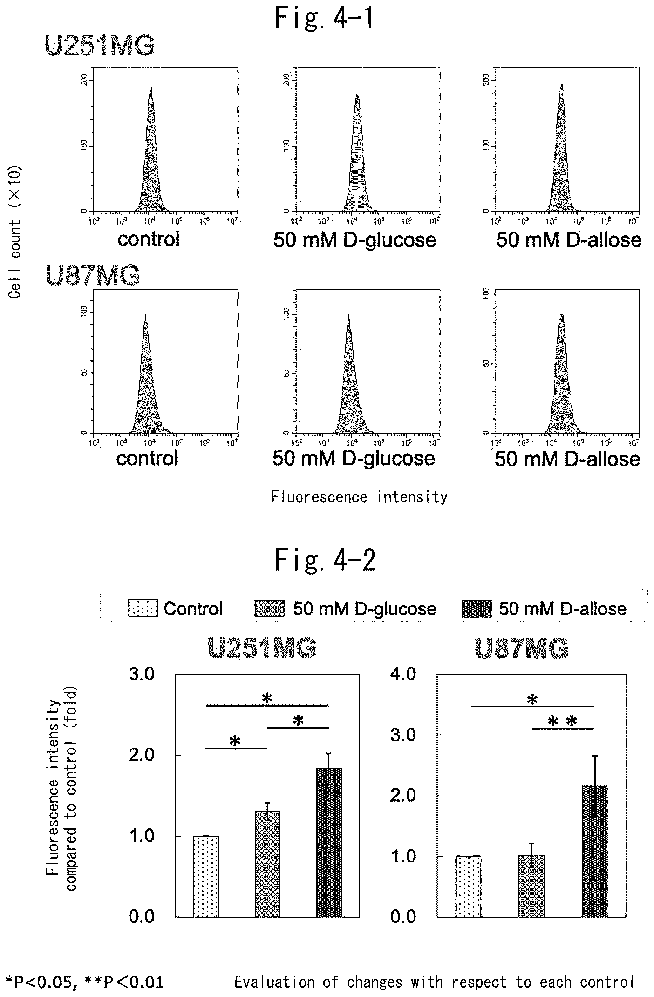

Next, CytoTell^{™}, a fluorogenic probe for flow cytometry which is recently being used as a cell proliferation monitoring reagent, was taken up into the brain tumor cells for 24 hours, and the cells were allowed to stand for 96 hours either without administration of a sugar (control) or with administration of 50 mM D-glucose and 50 mM D-allose, and attenuation of the fluorogenic probe intensity in the cells was evaluated (Fig. 4-1).

The results demonstrated that administration of 50 mM D-allose which induces a growth inhibition effect in brain tumor cells notably inhibited attenuation of fluorescent probe intensity in the brain tumor cells (Fig. 4-2).

The results indicated that D-allose has an effect of delaying the proliferation speed of cells without causing cell death by apoptosis or cell cycle arrest. Since this strongly suggests that it is possible to produce "cell slow-cycling" whereby the cell cycle is generally slowed without arrest, further analysis was carried out using the cultured human pancreatic cancer cell line PK-1.

### <Example 2: Analysis using cultured human pancreatic cancer cells>

PK-1 cells, as cultured human pancreatic cancer cells, were cultured for 48 hours with addition of D-allose or D-glucose at 25 or 50 mM concentration, and then analyzed by WST-1 assay to compare the effects on cell proliferation.

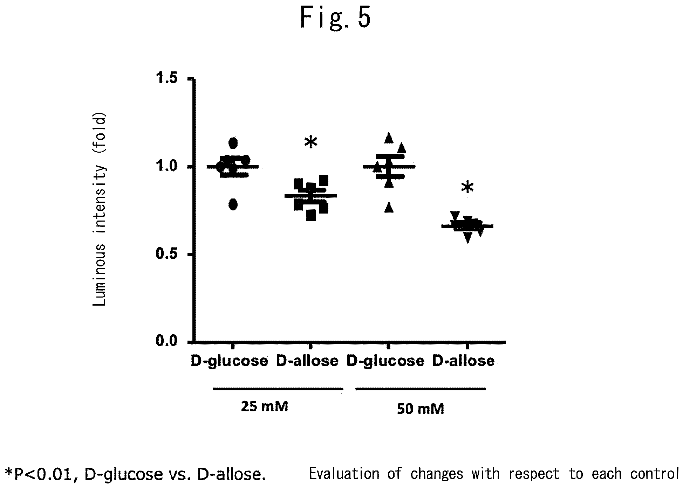

Similar to the results obtained with cultured brain tumor cells, D-allose exhibited a cell growth inhibitory effect on the PK-1 cells as a cultured pancreatic cancer cell line, as shown in Fig. 5.

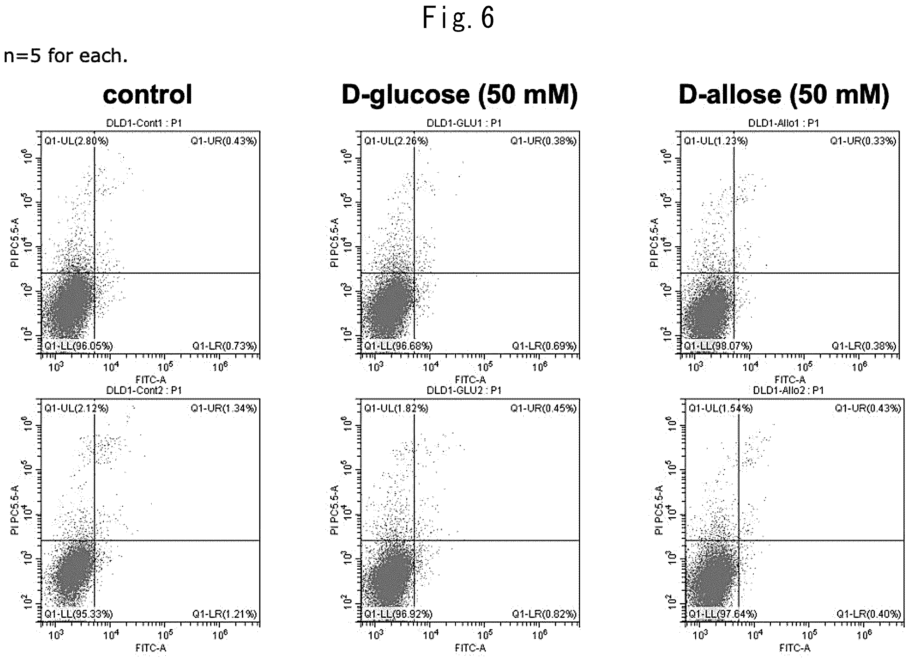

Apoptosis was also evaluated by flow cytometry with annexin V staining, and it was confirmed that apoptosis was not produced by D-allose at a dose of 50 mM which induced a growth inhibition effect on PK-1 pancreatic cancer cells (Fig. 6).

Considering the possibility that the growth inhibition effect on cultured human PK-1 pancreatic cancer cells by D-allose may be via arrest of the cell cycle, changes in the cell cycle were identified using flow cytometry (FACS analysis). Specifically, PK-1 cells were brought to a serum-starved state overnight and then fixed with 70% ethanol overnight at -20°C. For staining of the cellular DNA, the cells obtained as a centrifuged pellet were resuspended in 450 µL of propidium iodide (PI)/RNase solution (Cell Signaling Technology, Danvers, MA, USA), and cultured in a dark environment at room temperature for 30 minutes. Analysis was then carried out with a flow cytometer (Cytomics FC500 flow cytometry system, Beckman Coulter, Indianapolis, IN, USA) using 10,000 cells per sample.

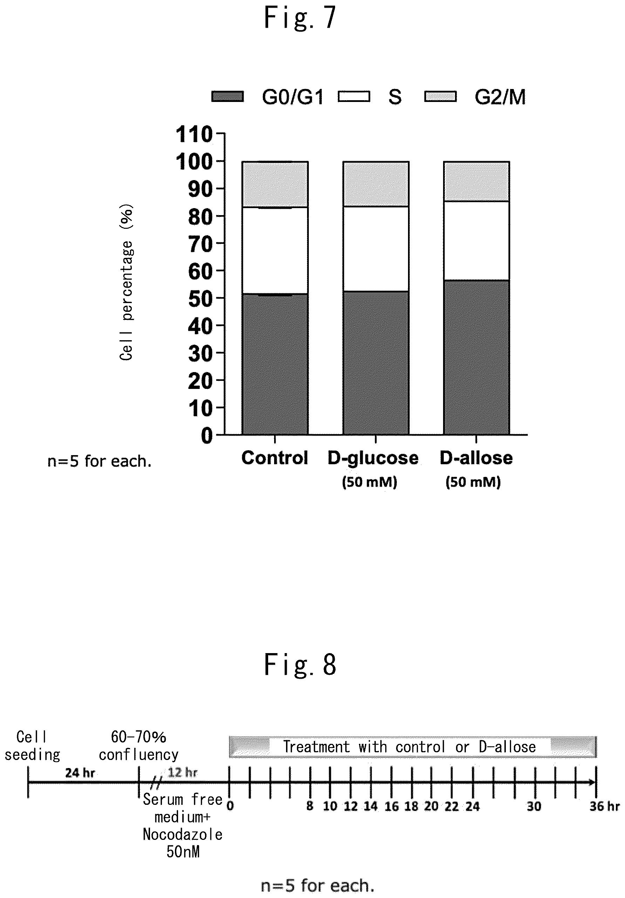

As a result, a slight increase was found in the G0/G1 phase ratio of PK-1 pancreatic cancer cells with administration of 50 mM D-allose which notably inhibits proliferation of PK-1 pancreatic cancer cells (Fig. 7).

However, doubts remained regarding whether this level of change by D-allose could have produced such a strong growth inhibition effect. Since it was therefore conjectured that D-allose may generally delay changes in the cell cycle, the verification experiment schematically illustrated in Fig. 8 was carried out.

First, 50 nM of nocodazole was reacted with cultured human PK-1 pancreatic cancer cells for 12 hours to preemptively stop the cell cycle. The nocodazole was then removed from the culture solution to begin restart of the cell cycle, thus allowing periodic observation of the cell cycle from a reset state. The effect of D-allose on the speed of the cell cycle was investigated in detail under these experimental conditions.

As a result, the cultured human PK-1 pancreatic cancer cells that were administered D-allose clearly had significant delaying of transition from the G0/1 phase to the S phase. This delaying effect was also exhibited for transition to the G2/M phase as a result.

It was therefore thought that D-allose might be linked with an effect of delaying the overall speed of the cell cycle and inhibiting proliferation, by an action of significantly delaying transition from the G0/1 phase to the S phase without arresting the cell cycle.

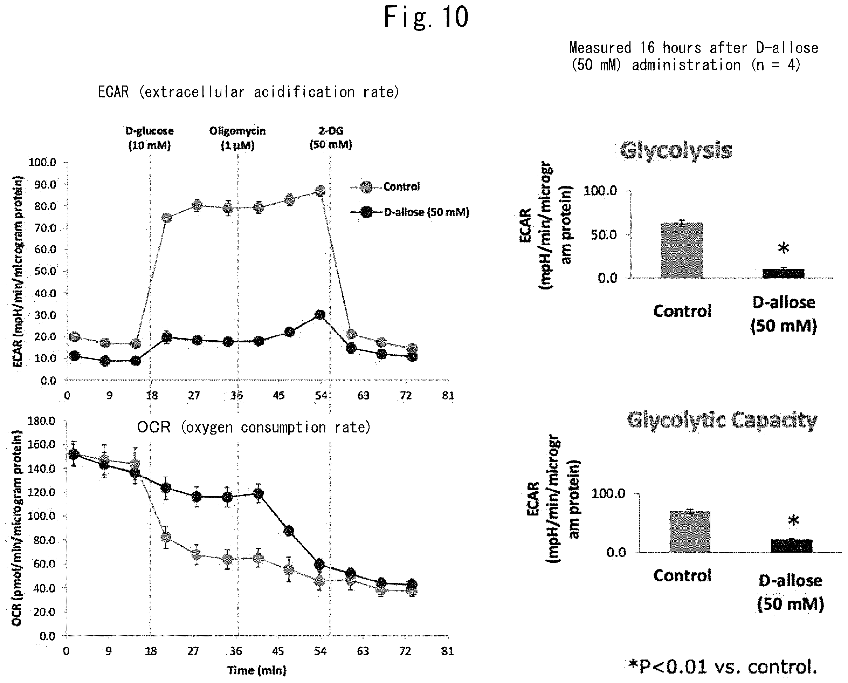

Since the reason for the effect exhibited by D-allose has not been elucidated, an XFe24 extracellular flux analyzer (24-well) by Agilent Technologies (previously Seahorse Bioscience) was used to measure the oxygen consumption rate (OCR value) and extracellular acidification rate (ECAR value) of cultured human PK-1 pancreatic cancer cells and examine effects on the glycolysis (Fig. 10).

As a result it was shown that administration of D-allose to cultured human PK-1 pancreatic cancer cells at 50 mM concentration for 16 hours notably inhibited the glycolysis in the cells. It was further shown that the "glycolytic capacity" representing the maximum function of the glycolysis in the cells, or its reserve capacity, was lowered as well.

### <Example 3: Analysis using cultured human pancreatic cancer cells>

In order to confirm that D-allose derivatives also have a similar effect of lengthening the period in which cells remain in the G0/1 phase of the cell cycle, the D-allose derivative allitol was used for an experiment in the same manner as Example 2. The sugar alcohol D-mannitol was used as a control.

PK-1 cells, as cultured human pancreatic cancer cells, were cultured for 48 hours with addition of allitol or D-mannitol at 50 mM concentration, and then analyzed by WST-1 assay to compare the effects on cell proliferation.

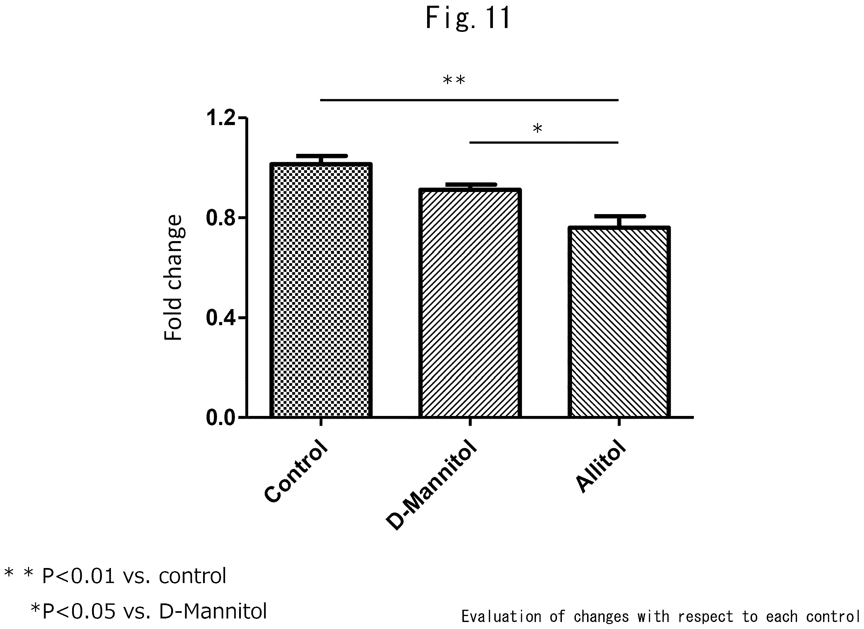

Allitol exhibited a cell proliferation inhibiting effect in the cultured PK-1 pancreatic cancer cells, similar to D-allose (Fig. 11).

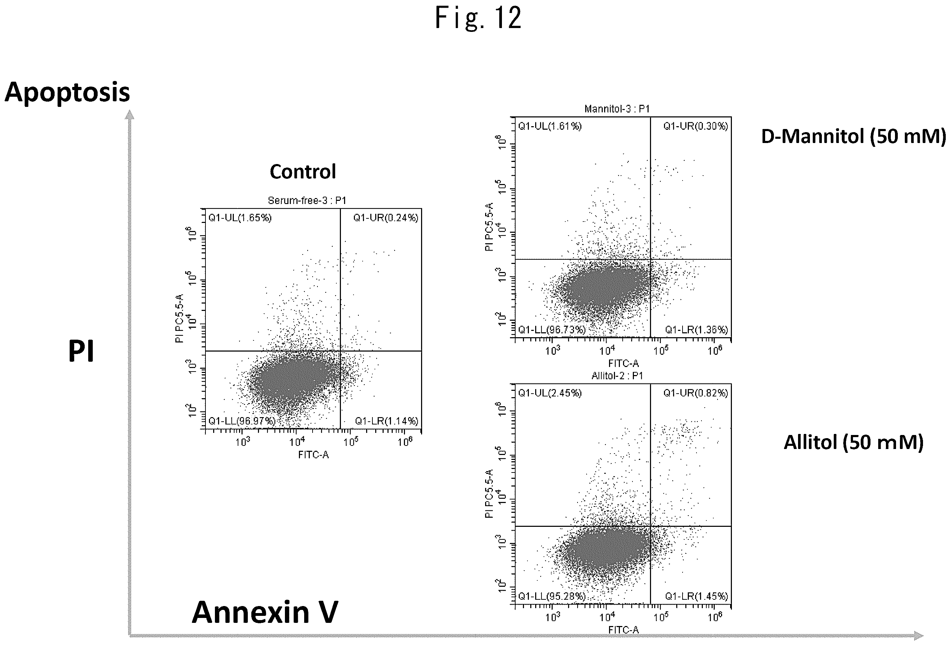

Apoptosis was evaluated by flow cytometry using annexin V staining. As a result, it was confirmed that no apoptosis had occurred with allitol at a concentration of 50 mM which exhibited a growth inhibition effect on PK-1 pancreatic cancer cells (Fig. 12).

Considering the possibility that the growth inhibition effect on cultured human PK-1 pancreatic cancer cells by allitol may be via arrest of the cell cycle, changes in the cell cycle were identified using flow cytometry (FACS analysis) by the same method as Example 2.

As a result, it became clear that allitol also significantly delayed the transition from the G0/1 phase to the S phase in cultured human PK-1 pancreatic cancer cells, similar to administering D-allose, although the effect was weaker than with D-allose. This delaying effect was also exhibited for transition to the G2/M phase as a result.

This strongly suggested the possibility that, similar to D-allose, allitol as well may have an effect of delaying the overall speed of the cell cycle and inhibiting cell proliferation, by an action of significantly delaying transition from the G0/1 phase to the S phase without arresting the cell cycle.

In the same manner as Example 2, an XFe24 extracellular flux analyzer (24-well) by Agilent Technologies (previously Seahorse Bioscience) was used to measure the oxygen consumption rate (OCR value) and extracellular acidification rate (ECAR value) of cultured human PK-1 pancreatic cancer cells and examine effects on the glycolysis.

As a result it was shown that administration of allitol to cultured human PK-1 pancreatic cancer cells at 50 mM concentration for 18 hours inhibited the glycolysis in the cells. It was further shown that the "glycolytic capacity" representing the maximum function of the glycolysis in the cells, or its reserve capacity, was significantly lowered as well.

The experimental data obtained thus far suggest that D-allose and allitol significantly delayed the transition from the G0/1 phase to the S phase by inhibiting the glycolysis, a mechanism associated with "cell slow-cycling", and thus produce inhibitory action on cancer cells.

### <Example 4: Metabolome analysis using human pancreatic cancer cell line>

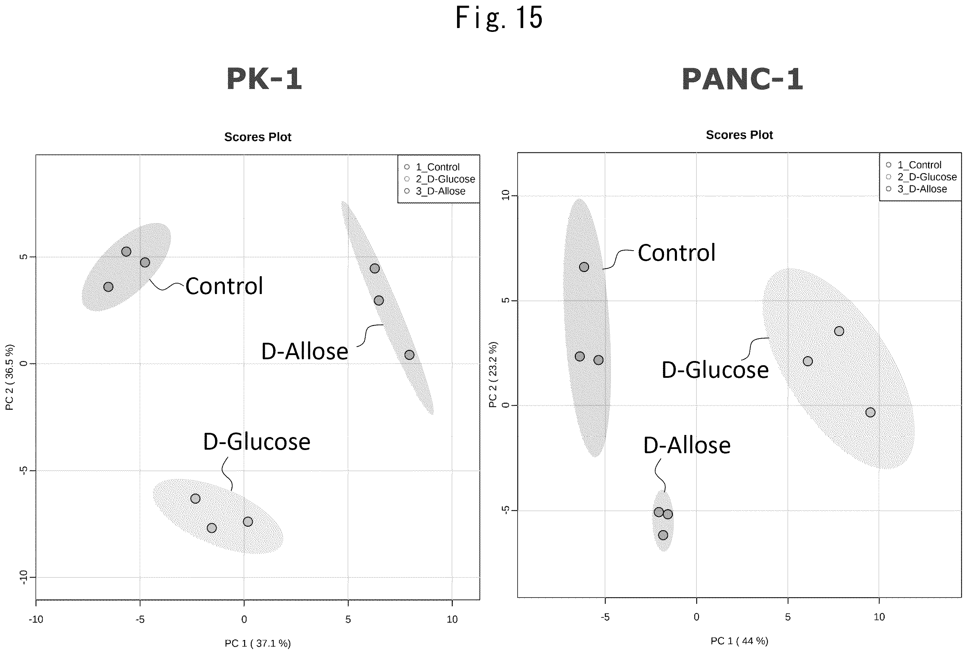

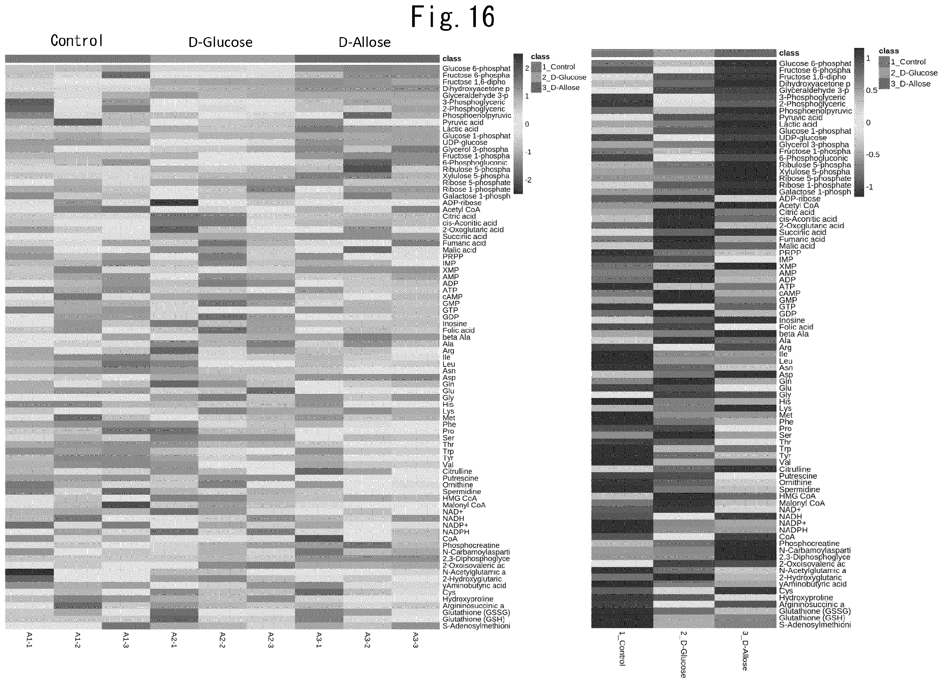

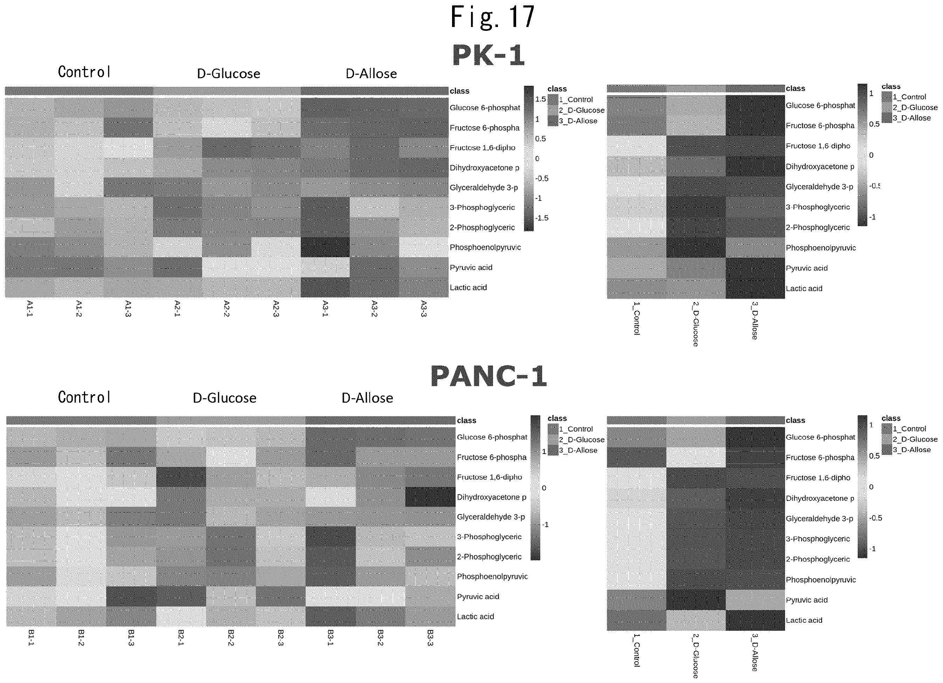

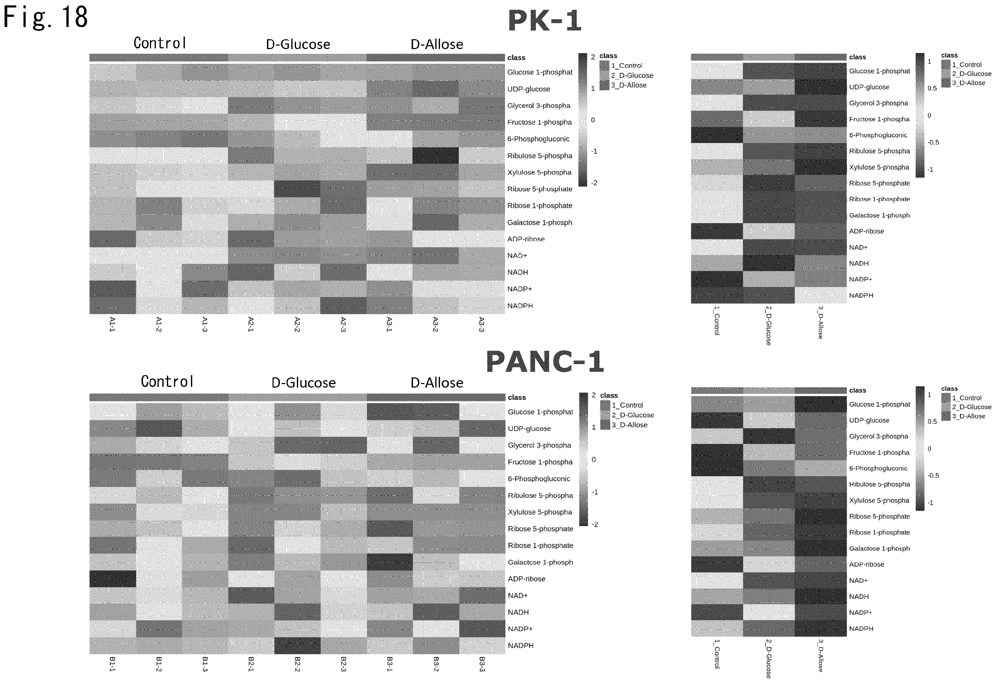

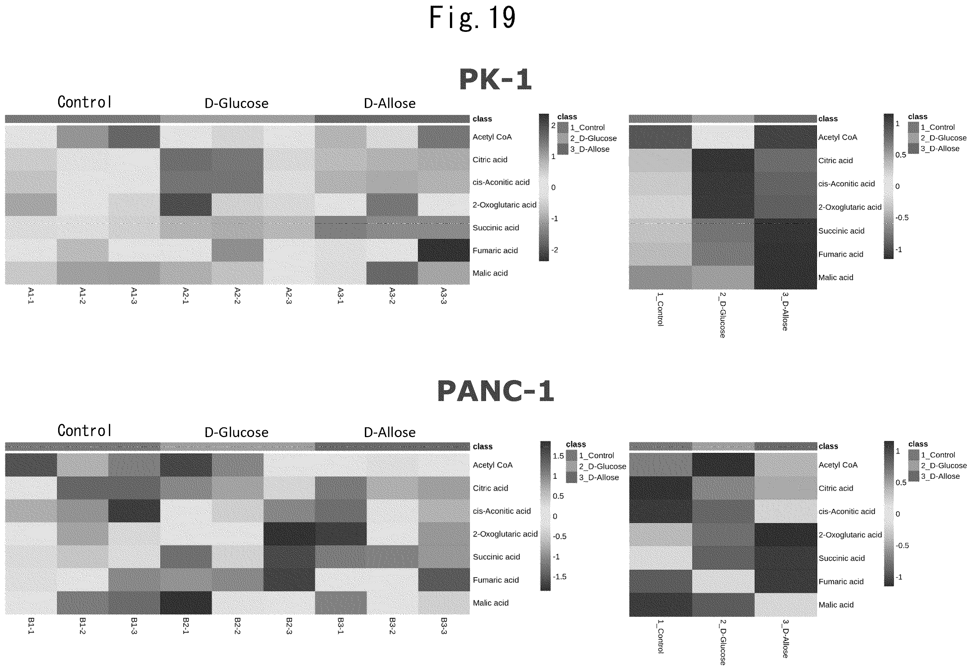

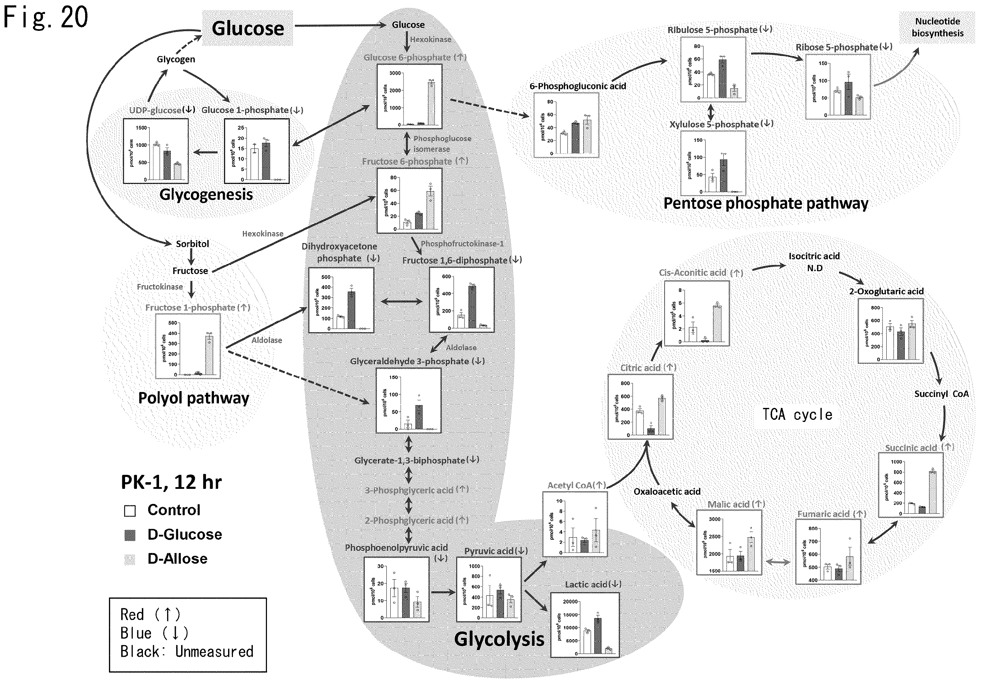

Cultured human pancreatic cancer cells PK-1 and PANC-1 (5.0 × 10⁶) were cultured for 12 hours at 37°C in a 5% CO₂ atmosphere, either without addition of a sugar (control) or with addition of 50 mM D-glucose or D-allose. Samples of both cells were then prepared according to the protocol provided by Human Metabolome Technologies, Inc. (Tsuruoka City, Yamagata Prefecture, Japan) and measured for 116 substances (see Table 1) related to central energy metabolism (glycolysis, TCA cycle, amino acid metabolism and nucleic acid metabolism) using an Analysis Service provided by the same company (C-SCOPE (https://humanmetabolome.com/jpn/service/analysis/cscope/)), after which principal component analysis (PCA) (Fig. 15), heat map analysis (Figs. 16 to 19) and pathway map analysis (Fig. 20) were carried out based on the measurement results.

**[Table 1]**

| Central carbon metabolism | Purine metabolism | Amino acid | Other |
|---|---|---|---|
| Acetyl CoA | Adenine | Ala | Acetoacetyl CoA |
| cis-Aconitic acid | Adenosine | Arg | *N*-Acetylglutamic acid |
| Argininosuccinic acid | Adenylosuccinic acid | Asn | S-Adenosylhomocysteine |
| Citric acid | ADP | Asp | S-Adenosylmethionine |
| CoA | ADP-rlbote | Cys | β-Ala |
| Dihydroxyacetone phosphate | AMP | Gln | γ-Aminobutyric acid |
| 2,3-Diphosphoglycericacid | ATP | Glu | Betaine |
| Erythrose 4-phosphate | cAMP | Gly | Betaine aldehyde |
| Fructose 1,6-diphosphate | cGMP | His | *N*-Carbamoylaspartic acid |
| Fructose 1-phosphate | GDP | Ile | Carbamoylphosphate |
| Fructose 6-phosphate | GMP | Leu | Carnitine |
| Fumaric acid | GTP | Lys | Carnosine |
| Galactose 1-phosphate | Guanine | Met | Choline |
| Glucose 1-phosphate | Guanosine | Phe | Citrulline |
| Glucose 6-phosphate | Hypoxanthine | Pro | Creatine |
| Glyceraldehyde 3-phosphate | IMP | Ser | Creatinine |
| Glycerol 3-phosphate | Inosine | Thr | Cystathionine |
| Glycolic acid | Uric acid | Trp | *N*,*N*-Dimethylglycine |
| Glyoxylic acid | Xanthine | Tyr | Folic acid |
| 2-Hydroxyglutaric acid | XMP | Val | Glutathione (GSH) |
| Isocitric acid | | | Glutathione (GSSG) |
| Lactic acid | | | HMG CoA |
| Malic acid | | | Homocysteine |
| 2-Oxoglutaric acid | | | Homoserine |
| 2-Oxoisovaleric acid | | | Hydroxyproline |
| Phosphoenolpyruvic acid | | | Malonyl CoA |
| 2-Phosphoglyceric acid | | | Mevalonic acid |
| 3-Phosphoglyceric acid | | | NAD* |
| 6-Phosphogluconic acid | | | NADH |
| PRPP | | | NADP* |
| Pyruvic acid | | | NADPH |
| Ribose 1-phosphate | | | Ornithine |
| Ribose 5-phosphate | | | Phosphocreatine |
| Ribulose 5-phosphate | | | Putrescine |
| Sedoheptulose 7-phosphate | | | Sarcosine |
| Succinic acid | | | Spermidine |
| UDP-glucose | | | Spermine |
| Xylulose 5-phosphate | | | Urea |

The results of principal component analysis (Fig. 15) and heat map analysis (Figs. 16 to 19) clearly showed that pancreatic cancer cells have a significantly altered intracellular metabolic profile after addition of D-allose. In particular, addition of D-allose to pancreatic cancer cells increased glucose 6-phosphate and fructose 6-phosphate in the glycolysis while reducing fructose 1,6-diphosphate. Addition of D-allose also increased fructose 1-phosphate in the polyol pathway. These results suggested that D-allose may act on routes that mediate the metabolism of these substances in the glycolysis and the polyol pathway (Fig. 20).

### <Example 5: Visualizing cell cycle of human pancreatic cancer cell line PK-1 with addition of D-allose>

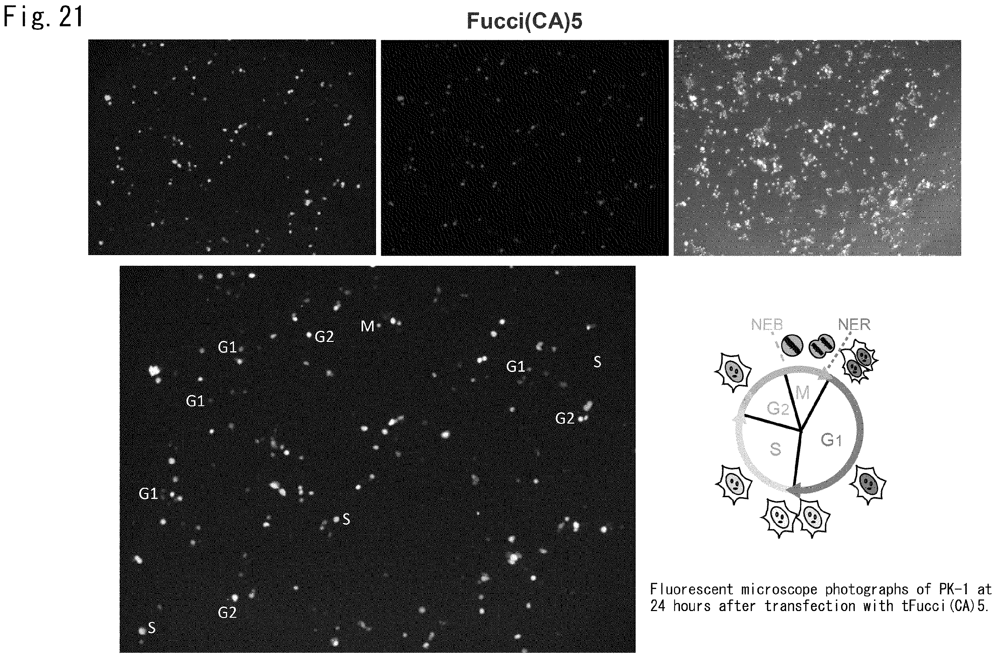

The human pancreatic cancer cell line PK-1 was transfected with tFucci(CA)5 (Plasmid #153521) (see Ando R., et al., Two new coral fluorescent proteins of distinct colors for sharp visualization of cell-cycle progression. bioRxiv, 2020, https://www.biorxiv.org/content/10.1 101/2020.03.30.015156v2) purchased from Addgene (Massachusetts, USA), for cell cycle visualization. The tFucci(CA)5-transfected cells were observed with a fluorescent microscope and exhibited red in the G1 phase, green in the S phase and yellow-orange in the G2/M phase (Fig. 21). The cells were used to observe effects on the cell cycle of the human pancreatic cancer cell line PK-1 with and without addition of D-allose (50 mM).

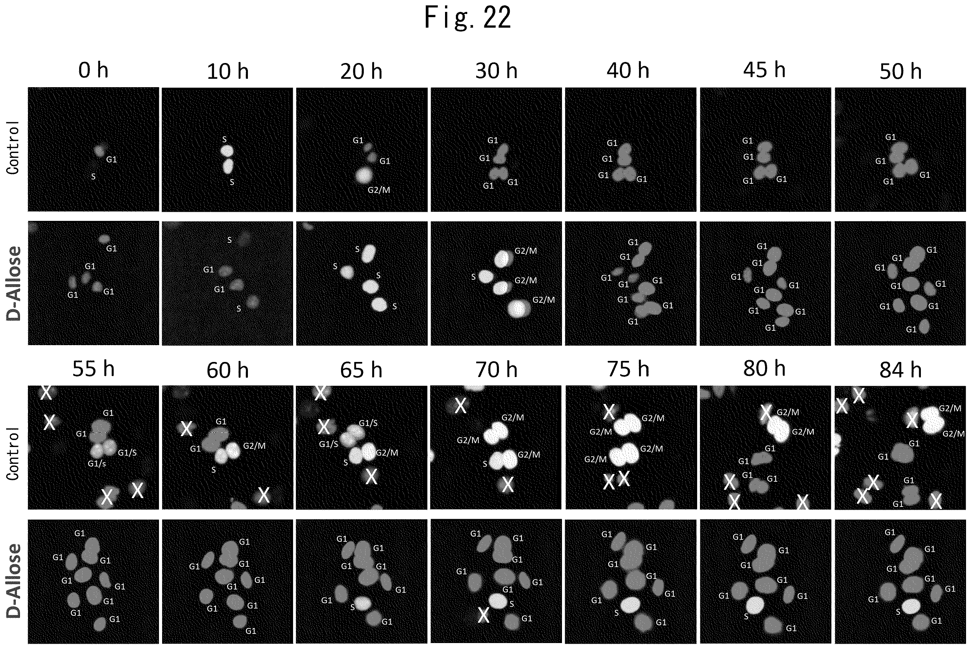

As a result, addition of D-allose was shown to delay the cell cycle, primarily at transition from the G1 phase to the S phase of the human pancreatic cancer cell line PK-1 (Fig. 22).

## Claims

1. An agent for delaying cell proliferation, comprising D-allose and/or a derivative thereof, and/or a mixture thereof as an active ingredient, wherein the agent lengthens the period in which cells remain in the G0/1 phase of the cell cycle.

2. The agent for delaying cell proliferation according to claim 1, wherein the D-allose derivative is allitol.

3. The agent for delaying cell proliferation according to claim 1 or 2, which does not arrest the cell cycle.

4. The agent for delaying cell proliferation according to any one of claims 1 to 3, which inhibits activity of the glycolysis in cells.

5. The agent for delaying cell proliferation according to any one of claims 1 to 4, which delays proliferation of cancer cells.

6. The agent for delaying cell proliferation according to any one of claims 1 to 5, which delays proliferation of pancreatic cancer cells or brain tumor cells.

7. The agent for delaying cell proliferation according to any one of claims 1 to 4, which delays senescence of cells.

8. A cosmetic for delaying senescence of cells, comprising an agent for delaying cell proliferation according to any one of claims 1 to 4.

9. A method for delaying cell proliferation in a subject in need thereof, comprising administrating an agent for delaying cell proliferation comprising D-allose and/or a derivative thereof, and/or a mixture thereof as an active ingredient, to the subject, to lengthen the period in which cells remain in the G0/1 phase of the cell cycle.
